(19) Europäisches Patentamt
European Patent Office
Office européen des brevets

(11) **EP 2 196 171 A1**

(12) **EUROPEAN PATENT APPLICATION**

(43) Date of publication:
**16.06.2010 Bulletin 2010/24**

(51) Int Cl.:
*A61F 2/02* *(2006.01)*        *A61L 27/16* *(2006.01)*
*A61L 27/34* *(2006.01)*        *C08J 7/18* *(2006.01)*

(21) Application number: **09178527.9**

(22) Date of filing: **09.12.2009**

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**
Designated Extension States:
**AL BA RS**

(30) Priority: **10.12.2008 HU 0800753**

(71) Applicant: **METRIMED Orvosi Müszergyártó Kft.
6800 Hódmezövásárhely (HU)**

(72) Inventors:
• **Blaskovics, Ferenc
6727, Szeged (HU)**

• **Venglovecz, Erzsébet
6800, Hódmezövásárhely (HU)**
• **Zsoldos, Gabriella
1046, Budapest (HU)**
• **Marossy, Kálmán
3700, Kiserdö sor 39. (HU)**
• **Czél, György
3525, Cserhát u. 11. (HU)**

(74) Representative: **Fehérvari, Flora
DANUBIA Patent and Law Office LLC
POB 198.
1368 Budapest (HU)**

(54) **Wear resistant gradient polymeric material and process for the preparation of the same**

(57)    The invention is related to a wear resistant gradient polymeric material having an inner phase include a first polymer and an outer phase including the first polymer mixed with a second polymer, in which the chains of the first and the second polymer form an interpenetrating network, and the ratio of the second polymer decreases from the surface of the outer phase in the direction of the inner phase, further, both phases of the polymeric material are cross-linked. The invention also encompasses the preparation method of the above polymeric material.

The inventive polymeric material shows high wear resistance, further, it has high resistance against physical and chemical impacts. The lifetime of an orthopaedic prosthesis prepared from the polymeric material according to the invention is long and it provides strong attachment to the bone cement.

## Description

TECHNICAL FIELD OF THE INVENTION

The present invention relates to a wear resistant gradient polymeric material having an inner phase including a first polymer and an outer phase including said first polymer and a second polymer, in which the chains of the second polymer are penetrated through the chains of the first polymer, and the ratio of the second polymer to the first polymer decreases from the surface of the outer phase in the direction of the inner phase, furthermore the polymer chains in both phases are connected via covalent bonds to each other. The inventive polymer material shows better wear resistance as compared to the conventional polymers. The invention also encompasses the preparation of the aforementioned polymeric material and its application as a structural material for articles having surfaces subjected to high wearing stress such as medical prosthetic members or plastic mechanical parts.

BACKGROUND OF THE INVENTION

During the past decades, plastics significantly gained importance in a broad range of technical fields and nowadays they are widely used to replace different conventional materials due to their usually better technical properties and lower cost.

The basic requirements for the applicability of polymers are proper mechanical properties and long lifetime. Wear resistant plastic materials are often used as structural materials for moving mechanical parts with gliding surfaces such as plain bearings or cogwheels and also for toys and sport goods made of plastic such as different disks and balls, e.g., golf ball.

Another important field of application of polymer materials is the restoration of injuries of accidental origin and joints damaged due to various illnesses, in which orthopaedic surgeons and traumatologysts more and more often use endoprosthesises.

The major development in orthopaedic surgery in the 20th century probably was the replacement of damaged hip and knee joints (furthermore, shoulder, elbow, ankle, wrist, and finger joints) by using full joint endoprosthesis (full arthroplastics).

The first efforts have already been made at the beginning of the 20th century to replace bones by artificial materials. The first significant developments however are dated back only to 1954, when Sir John Chanley - who made pioneering work on the field of hip prosthesis - started his developments in England. The development and manufacturing of endoprosthesis have been going on also in Hungary since several decades.

Several methods exist for implanting joint prosthesis. The most simple and also the most widely used method is their implantation using bone cement. As bone cement, usually a cross-linking polymer, such as poly(methyl methacrylate) is used, which is usually prepared by mixing two components during the surgery.

Concerning mechanical parts with gliding surfaces the major challenge is to decrease the wearing, which can be achieved by forming gliding surfaces as smooth as possible. Another approach to the development is to increase the wear resistance of mechanical parts made of polymeric material. Such plastic raw materials with outstanding properties, which have been developed for medical implants are the Ultra High Molecular Weight Polyethylene (UHMWPE), poly (ether ether ketone) (PEEK), and other partially amorphous, partially crystalline polymers.

In the last forty years UHMWPE has been widely used as raw material for joint prosthesis. Although UHMWPE is successfully applied, its lifetime is limited. Wearing and damage of UHMWPE often make the change of the prosthesis, i.e. the reoperation necessary. The abrasion of the acetabular cup can reach the millimeter size, and the implant may become loose and can cause pain to the patient.

Several methods exist for improving the gliding properties of the polymeric material. In DE 203 21 263 U1 silicon oil was injected between the gliding surfaces of an UHMWPE prosthesis for improving its gliding properties.

In EP 1 779 877 A1 beta-carotene was incorporated into the UHMWPE material and its improving effect on the wear resistance was confirmed.

US 6494917 describes a wear resistant implant, which was prepared by a surface-gradient cross-linking method. The cross-linking was carried out by electron radiation or chemical initiation in the surface layer of the polyethylene material so that the extent of cross-linking in the direction of the inner part of the polyethylene gradually decreased to zero. The remaining free radicals were eliminated by re-melting of the product.

In US 2007/0118227 and US 7186364, King and coworkers, describe a plastic prosthesis bearing consisting of two layers, which can well adhere to the bone cement. The inner layer of the prosthesis member was made of UHMWPE, whereas its outer layer was made of ethylene- acrylate copolymer, where the ethylene and acrylate components provide strong bonding to the polyethylene forming the inner layer and to the bone cement, respectively. In the above work the polymer layers are fixed together by molding and/or fusion. It is emphasized by the authors that irradiation (gamma radiation) was not applied during the process.

US 5645594 describes a medical implant consisting of at least two layers, in which the first layer contacting the joint is prepared from UHMWPE, whereas the second layer contacting the bone is made of a mixture of UHMWPE and poly(methyl methacrylate) (PMMA), and optionally a third layer consisting PMMA is also applied. In the preparation of the second layer, dry granulates of the polymers are mixed and the layers are pressed together at the required temperature and, according to the description, the PMMA is melted and penetrates into the UHMWPE polymer. According to the authors the thus prepared prosthetic implant shows a better bonding to the bone cement, and has an improved stiffness and shows lower cold flow rates as compared to a prosthetic implant prepared from UHMWPE alone. The wear resistance of the prosthetic implant is not discussed in this document.

In the above technical solution the fused particles of PMMA and UHMWPE form islets in the PMMA containing layer, however, it follows from this process that the chains of the two different polymers are not mixed at a molecular level.

The aim of our research was to develop a polymer based gradient material which shows better wear resistance and has longer lifetime as compared to a conventional polymeric material, further, its other characteristics are also favorably modified, for instance, its adhesive properties are improved, so that it bonds more strongly to bone cement when applied in prosthesis members.

BRIEF DESCRIPTION OF THE INVENTION

The present invention is based on a finding that the functional properties of polymeric materials are considerably improved when an outer phase is formed on their surface, which includes a second polymer mixed with the polymer of the inner phase, wherein the chains of the second polymer are penetrated through the chains of the first polymer, and the ratio of the second polymer to the first polymer continuously decreases from the surface of the outer layer in the direction of the inner phase, further, both phases are cross-linked. Thus, a polymer based functional gradient material (FGM) is obtained.

Gradient material means an anisotropic material having a property, for example its composition, which continuously changes in the direction of at least one of its dimensions. In the two-component gradient material according to the present invention this anisotropy is manifested in that at a certain point of the material one of the components is present as a major component, and proceeding from this point towards an other point the ratio of the components is gradually shifted in favor of an other component, and in the second point this latter component becomes the major component.

In line with the above discussion one aspect of the present invention is a polymeric material having an inner phase including a first polymer and an outer phase including said first polymer and a second polymer, in which the chains of the second polymer are penetrated through the chains of the first polymer, and the ratio of the second polymer to the first polymer decreases from the surface of the outer phase in the direction of the inner phase, further, the polymer chains in both phases are connected via covalent bonds to each other.

A further aspect of the present invention is a wear resistant gradient prosthesis material having an inner phase including a first polymer and an outer phase including said first polymer and a second polymer, in which the chains of the second polymer are penetrated through the chains of the first polymer, and the ratio of the second polymer to the first polymer decreases from the surface of the outer phase in the direction of the inner phase, further, the polymer chains in both phases are connected via covalent bonds to each other.

Another aspect of the present invention is a prosthesis member including the above mentioned gradient polymeric material. A further aspect of the invention is a process for the production of the above polymeric material, in which the first polymer is contacted with the liquid monomer(s) forming the second polymer or a solution thereof and, when a constant weight is reached, the monomer(s) diffused into the first polymer are polymerized and cross-links are formed in the polymeric material of the prosthesis.

In preferred embodiments of the present invention UHWMPE is utilized as a first polymer.

In the preferred embodiments of the present invention the second polymer is selected from the polymers of allylic compounds, such as allyl esters and allyl ethers, or acrylic acid/methacrylic acid derivatives, or linear and cyclic dienes. In the most preferred embodiments the second polymer is a homopolymer or a copolymer including a monomer selected from the group of methyl methacrylate, diethylene glycol bis-allyl carbonate, ethylene glycol dimethacrylate, and triallyl cyanurate.

In one of the more preferred embodiment of the present invention the first polymer is UHWMPE and the second polymer is methyl methacrylate.

In a preferred embodiment of the invention the polymer and the monomer, depending on the utilized monomer(s), are contacted at a temperature between ambient temperature and 60°C for 1 to 336 hours in liquid phase monomer or monomer mixture, without solvent or optionally in a solution containing a solvent. According to a preferred embodiment of the inventive process the contacting step is carried out at 20°C to 30°C, or - if the melting point of the monomer is higher - at a temperature over its melting point, e.g. at 40°C to 60°C for at least 48 hours.

As a solvent, for example toluene, dioxane, diethyl ether or other suitable solvent is used.

In a preferred embodiment of the inventive process a prepolymerization step is carried out as a first step, then in a second step the polymeric material is treated with high-energy irradiation.

In an embodiment of the process according to the present invention the prepolymerization is carried out by heat treatment, UV radiation, or by using a chemical initiator, whereas the high-energy irradiation is performed using gamma radiation or electron- radiation.

In another embodiment of the process according to the present invention the polymerization and cross-linking are carried out in one step using high-energy irradiation.

DETAILED DESCRIPTION OF THE INVENTION

The gradient layer developed here is formed on the product brought to its final form, which can be an UHMWPE material developed for prosthesis production, however, other amorphous or partially crystalline polymeric materials for medical applications such as PEEK also can be applied, furthermore other widely used polymers can be applied for mechanical parts or toys.

During the development of the inventive process UHMWPE material widely used in orthopedics was applied, which has the following physical properties (according to ISO 5834/2):

| Characteristics | UHMWPE |
|---|---|
| Molar weight (g/mol) | $2 \cdot 10^6$ - $6 \cdot 10^6$ |
| Softening point (°C) | 125 - 138 |
| Density (g/cm$^3$) | 0.932 - 0.945 |
| Elasticity modulus (GPa) | 0.8 - 1.6 |
| Tensile strength (MPa) | 21 - 28 |
| Rupture strength (MPa) | 39-54 |
| Elongation (%) | 350 - 525 |
| Izod impact strength (J/m, at the notch, using a specimen with 3.175 mm thickness) | >1070 (no braking) |
| Crystallinity (%) | 39-75 |

The applied forms of the polymer are for example the following:

- product prepared from RAM extruded rod by cutting;
- product prepared by high pressure molding

In the process according to the invention the polymer product is immersed into a monomer containing liquid.

Concerning the applied monomers the requirements are the following:

- should be compatible with the amorphous phase of the polymer and can diffuse into it in a significant extent;
- can be polymerized easily;
- its purity is as high as possible;
- hard and highly stable in its polymerized form;
- biologically inactive in its polymerized form.

Monomers satisfying the above requirements are: methyl methacrylate, diethylene glycol bis-allyl carbonate, ethylene glycol dimethacrylate, and triallyl cianurate. Suitable mixtures of the monomers also can be used, which may result in the formation of a copolymer as a second polymer in the polymerization step.

The diffusion of the monomer is facilitated by soaking. The diffusion time during the soaking is 1 to 336 hours, preferably at least 48 hours, whereas the procedure can be carried out at any temperature compatible with the materials used, for example between ambient temperature and 60°C, for instance preferably at 20°C to 30°C when acrylates are used, and preferably at 40°C to 60°C when a monomer with a higher melting point, e.g. triallyl cyanurate is applied.

The soaking procedure can be carried out in one type of liquid monomer or liquid monomer mixture or in a monomer(s) dissolved in a solvent.

In the soaking step the monomer diffuses into the amorphous phase of the polymer and its weight increases in a small extent due to the diffusion.

The soaking procedure is carried out until constant weight is reached. Following the soaking step the monomer can be wiped off or can be left on the surface of the product. Then the polymerization and the cross-linking are carried out. As a first step of this latter procedure the monomer diffused into the product is subjected to prepolymerization.

The prepolymerization can be performed by using the following methods:

- thermal polymerization carried out in the temperature range of 20°C to 160°C for a time period necessary at least to reach complete polymerization in the entire volume;
- polymerization by UV radiation for a time period necessary at least to reach complete polymerization in the entire volume;
- the combination of the above two methods;
- polymerization using exclusively biologically neutral chemical initiator (e.g. lauryl peroxide).

In the next step high-energy radiation is applied.

In one embodiment the above described prepolymerization step can be omitted and the soaked product can be directly treated with high-energy irradiation.

The high-energy irradiation has multiple role. On the one hand, free radicals are formed in the material due to the high-energy irradiation, which can initiate polymerization processes. Thus, in the present process the polymerization of the monomer diffused into the polymer can be initiated by high-energy irradiation. On the other hand, covalent bonds can be formed between the polymer chains as an effect of the high-energy irradiation, i.e. cross-linking takes place. Cross-linking will significantly change the properties of the polymer; its mechanical strength, wear resistance, physical and chemical resistance, lifetime, and flow temperature increase, whereas its elasticity and swellability/solubility decreases. Moreover, high-energy irradiation is a widespread method for sterilization of medical tools and wrapped materials used in the medical practice.

Accordingly, the high-energy radiation used in the process according to the present invention has three main functions, namely the polymerization of the monomer diffused into the polymer and cross-linking of the polymeric material, during which from the monomer diffused into the UHMWPE material polymer chains are formed, which penetrate through the polyethylene chains and bond to them via covalent bonds; furthermore, in case of medical use the polymeric material can be also sterilized by high-energy irradiation.

As high-energy radiation gamma radiation (e.g. Co isotope) and electron or beta radiation can be applied; the absorbed dose is 1 to 1000 kGy, preferable up to 40 kGy, even more preferably about 20 kGy.

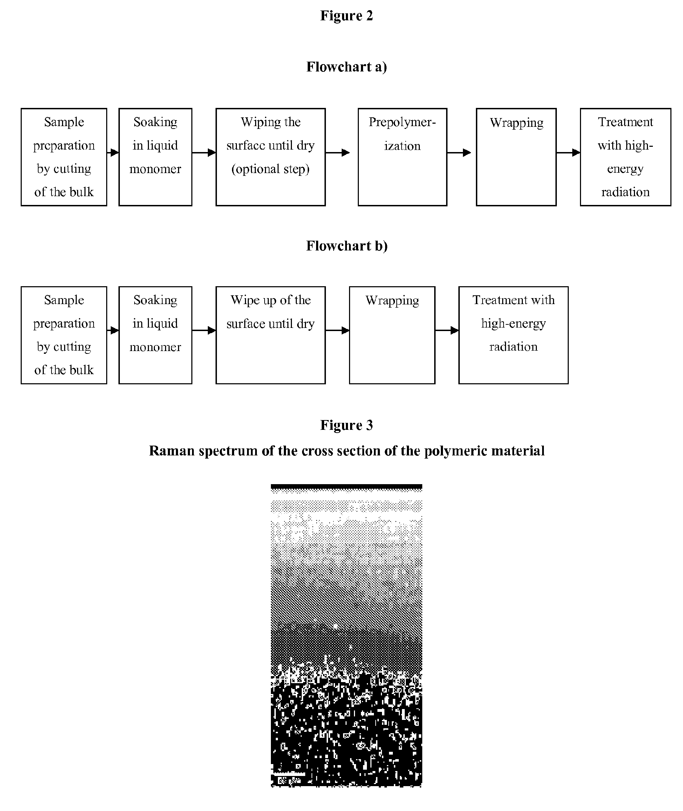

The flowcharts of the process for the preparation of the gradient polymeric material are shown in figure 2, where flowchart a) shows a process involving a prepolymerization step, while a process without a prepolymerization step is shown in flowchart b).

The properties of the above described polymeric material according to the present invention are significantly improved relatively to the first polymer applied, namely its mechanical strength, wear resistance and lifetime increase. Moreover, other characteristics of the polymeric material can be also favorably influenced. For example, when used in a medical prosthesis proper selection of the second polymer results in a product that can better adhere to the bone cement than the conventional UHMWPE material widely used in orthopaedic surgery. The gradient layer is preferably formed on the entire surface of the product, so that the risk of a loose prosthesis will decrease.

**Description of the figures**

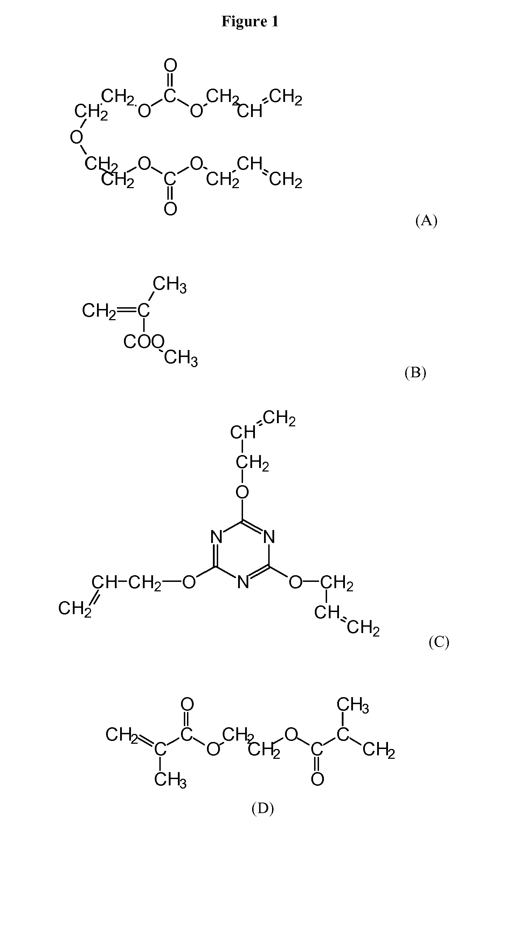

In Figure 1 the structure of diethylene glycol bis-allyl carbonate (A), methyl methacrylate (B), triallyl cyanurate (C), ethylene glycol dimethacrylate (D) monomers are shown;

Figure 2 illustrates flowcharts showing alternative methods for the preparation of the gradient polymeric material according to the present invention; flowchart a) illustrates a process including a prepolymerization step, whereas flowchart b) is related to a process comprising a single polymerization step carried out by high-energy irradiation;

Figure 3 shows the Raman spectrum of the cross section of the inventive polymeric material;

Figure 4 shows the composition curve obtained by the image analysis of the Raman spectrum of the inventive gradient polymeric material;

Figure 5 illustrates the changes of the abrasion coefficient observed during the wear test of each sample in the function of time.

**Examples**

**Example 1**

**Preparation of a gradient prosthesis material by treating the surface of UHMWPE starting material with diethylene glycol bis-allyl carbonate monomer**

A prosthesis members prepared from UHMWPE (GUR 1020) starting material having an inner diameter of 32 mm and an outer diameter of 49 mm with a geometry suitable for surgical implantation were placed into 300 ml of diethylene glycol bis-allyl carbonate (Acomon RAV 700) monomer in an exhauster box preferably away form light and were left there at 20°C to 30°C for 120 hours. They absorbed 0.026 mg/cm$^2$ monomer during this time. The members were then removed using forceps, the remaining monomer was wiped off their surface and finally the samples were wrapped airtight and treated with 21 kGy gamma radiation using Co isotope.

**Example 2**

**Preparation of a gradient prosthesis material by treating the surface of UHMWPE starting material with methyl methacrylate monomer**

A prosthesis members prepared from UHMWPE (GUR 1020) starting material having an inner diameter of 32 mm and an outer diameter of 49 mm with a geometry suitable for surgical implantation were placed into 300 ml of methyl methacrylate (Arkema Norsocryl) monomer in an exhauster box preferably away form light and were left there at 20°C to 30°C for 120 hours. They absorbed 0.489 mg/cm$^2$ monomer during this time. The members were then removed using forceps, the remaining monomer was wiped off their surface and finally the samples were wrapped airtight and treated with 21 kGy gamma radiation using Co isotope.

**Example 3**

**Preparation of a gradient prosthesis material by treating the surface of UHMWPE starting material with triallyl cyanurate monomer**

A prosthesis members prepared from UHMWPE (GUR 1020) starting material having an inner diameter of 32 mm and an outer diameter of 49 mm with a geometry suitable for surgical implantation were placed into 300 ml of triallyl cyanurate (Nanjing Yonghong Chemical Co., Ltd.) monomer in an exhauster box preferably away form light and were left there at 50°C to 60°C for 120 hours. They absorbed 0.001 mg/cm$^2$ monomer during this time. The members are then removed using forceps, the remaining monomer was wiped off their surface and finally the samples were wrapped airtight and treated with 21 kGy gamma radiation using Co isotope.

**Example 4**

**Preparation of a gradient prosthesis material by treating the surface of UHMWPE starting material with ethylene glycol dimethacrylate monomer**

A prosthesis members prepared from UHMWPE (GUR 1020) starting material having an inner diameter of 32 mm and an outer diameter of 49 mm with a geometry suitable for surgical implantation were placed into 300 ml of ethylene glycol dimethacrylate (Sartomer SR 206) monomer in an exhauster box preferably away form light and were left there at 20°C to 30°C for 120 hours. They absorbed 0.049 mg/cm$^2$ monomer during this time. The members were then removed using forceps, the remaining monomer was wiped off their surface and finally the samples were wrapped airtight and treated with 21 kGy gamma radiation using Co isotope.

**Example 5**

**Preparation of a gradient polymeric material by treating the surface of the UHMWPE starting material with methyl methacrylate monomer**

Disks prepared from UHMWPE (GUR 1020) starting material having a diameter of 50 mm and a height of 25 mm were placed into 300 ml of methyl methacrylate (Arkema Norsocryl) monomer in an exhauster box preferably away form light and were left there at 20°C to 30°C for 120 hours. They absorbed 0.467 mg/cm$^2$ monomer during this time.

The members were then removed using forceps, the remaining monomer was wiped off their surface and finally the samples were wrapped airtight and treated with 20 - 40 kGy electron radiation.

Studies of the physical properties of gradient polymeric materials

*Spectroscopic analysis*

In Figure 3 the depth analysis results obtained using a WiTec CRM 200 confocal Raman microscope with (red) laser at a wavelength of 632 nm on the sample prepared by the process according to Example 2 are shown.

In the upper part of the image the acrylate located in the outer phase of the gradient material appears in white color and in the lower part of the image the UHMWPE matrix forming the inner phase of the material is shown in black color, whereas proceeding from the top to the bottom between the two parts darker and darker shades of grey can be observed, which indicate the continuous change in the composition of the polymeric material from high acrylate content to high polyethylene content.

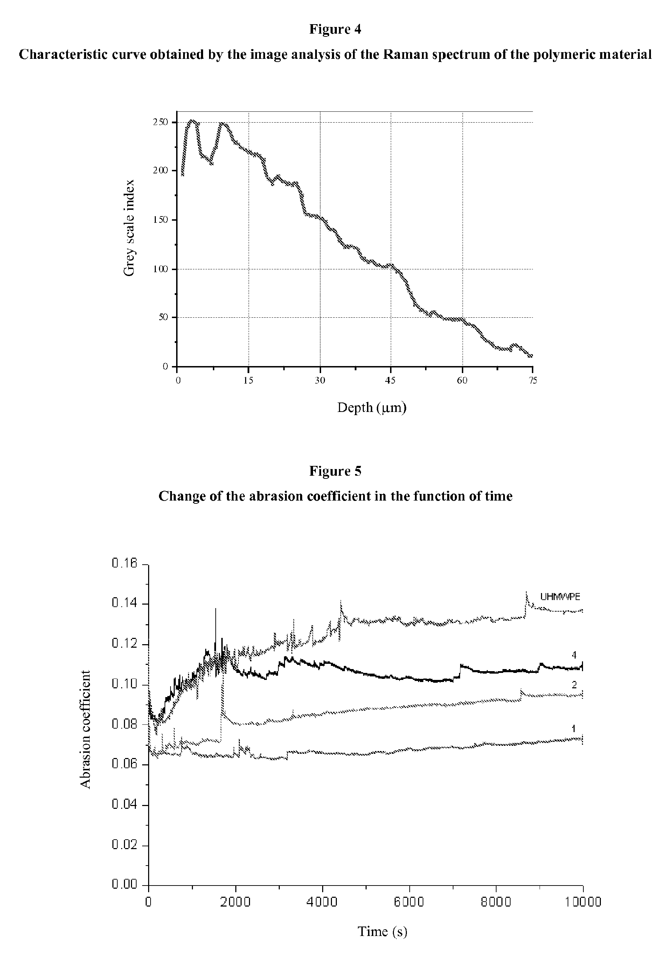

In Figure 4 the results of the image analysis are summarized, which show the grey scale index in the function of depth measured from the surface of the material; the diagram clearly indicates the continuous change of the polymer composition.

The above spectroscopic results readily confirm the gradient structure of the inventive material.

*Wear test of the gradient polymeric material*

The wear tests on the above samples according to Example 1, 2, and 4 were carried out at the University of Miskolc, Hungary using a CSEM tribometer in a "pin-on-disk" arrangement.

In the pin-on-disk tribology tests a contrast material is pressed by a constant tangential (circumferential) force on the surface of the rotated specimen (disk). The friction force between the two materials is measured in the function of the full abrasion path. The shape of the abrasion trace formed during the wear test is also studied. For this latter test profilometry is applied, that is, the shape of the abrasion trace is measured with high precision in the function of the abrasion path.

The apparatus, that is, the tribometer continuously records the friction force between the two materials during the wear test. The loading normal force is constant due to the weight load. The momentary abrasion coefficient can be calculated by the computer program of the apparatus. Following the test the amount of the material worn off the surface of the sample can be measured with a so called profilometer by using surface analytical methods.

From the amount of the worn off material and absorbed energy due to friction during the wear test the abrasion coefficient of the material can be determined, which is equal to the energy necessary for wearing off a unit volume.

$$\eta = \frac{V_W}{W_{absorbed}}; \quad [\eta] = \frac{mm^3}{MJ},$$

where
$\eta$ : abrasion coefficient;
$V_w$: worn off volume;
$W_{absorbed}$: absorbed energy due to friction.

The experimental parameters were the following:

- Circumferential force: $F_T$ = 10 N
- Abrasion time: t = 10 000 s = 2.78 h
- Circumferential speed: v = 10 cm s$^{-1}$
- Sampling frequency: f = 2 Hz

The changes in the abrasion coefficient of the samples during the measurement are illustrated in Figure 5. The experimental results are shown below in Table 1 in comparison with the results obtained for a UHMWPE material, where samples 1, 2, and 4 represent the polymer samples prepared in the corresponding examples and as the UHMWPE material a prosthesis member formed by a conventional method was used (i.e. prepared from GUR 1020, diameter 50 mm, height 25 mm, treated with 21 kGy gamma radiation).

**Table 1**

| | Abrasion coefficient ($mm^3$/MJ) | Worn off volume ($mm^3$) |
|---|---|---|
| UHMWPE | 50.9 | 0.51 |
| 1. | 30.5 | 0.31 |
| 2. | 32.4 | 0.32 |
| 3. | 33.3 | 0.33 |

The above results indicate that both the abrasion coefficient and the worn off volume are smaller for the three inventive gradient materials than for the UHMWPE material not treated by the inventive process. Thus the gradient materials according to the present invention show better wear properties than the conventional UHMWPE material.

*Study of the adhesion properties of the gradient polymeric material*

The adhesion of a gradient material according to the present invention and a conventional UHMWPE material to an acrylate bone cement widely used in prosthetics (Palacos-R) were subjected to tensile test using an Instron type universal material testing apparatus, in which two half of the standard specimens with a thickness of 1 mm were cut out from a conventional UHMWPE material or from the inventive material and each was fixed to a 2 - 3 mm thick bone cement layer with 10 mm x 10 mm overlapping. Results are summarized below in Table 2.

**Table 2**

| Results of the adhesion tests obtained with conventional UHMWPE and treated UHMWPE using bone cement | | |
|---|---|---|
| | Tensile strength (MPa) | Deformation (%) |
| UHMWPE | 0.372 | 0.20 |
| Sample of Ex. 2. | 0.675 | 0.35 |

The above results indicate that the adhesion of the bone cement to the surface of the polymeric material according to the present invention is twice as good.

Summary of the advantageous effects of the technical solution according to the present invention

The polymeric material prepared from the first and second polymer according to the present invention is harder and shows higher wear resistance than conventional polymers, the surface of which were not treated with the inventive method. The gradient structure of the polymeric material provides extremely strong bond between the two types of polymers, which eliminate the risk of the detachment of the wear resistant layer. Furthermore, its adhesion properties are better, for instance when used as a prosthesis member, its adhesion to the bone cement is stronger than that of the conventional UHMWPE material widely used as starting material for prosthesis. The surface treatment carried out according to the inventive process is simple, can be accomplished in 2 to 3 steps, while deformation of the product cannot be observed during the process, furthermore subsequent forming is not necessary.

**Claims**

1. A gradient polymeric material having an inner phase including a first polymer and an outer phase including said first polymer mixed with a second polymer, in which the chains of the second polymer are penetrated throgh the chains of the first polymer, and the ratio of the second polymer to the first polymer continuously decreases from the surface of the outer phase in the direction of the inner phase, further, the polymer chains in both phases are connected via covalent bonds to each other.

2. A gradient polymeric material according to claim 1, in which the first polymer is ultra high molecular weight polyethylene.

3.  A gradient polymeric material according to claim 1 or 2, in which the second polymer is a homopolymer or copolymer including monomers selected from the group of methyl methacrylate, diethylene glycol bis-allyl carbonate, ethylene glycol dimethacrylate, and triallyl cyanurate.

4.  A prosthesis member including a gradient polymeric material according to any one of claims 1 to 3.

5.  A process for the preparation of a gradient polymeric material according to any one of the forgoing claims 1 to 3, **characterized in that** the first polymer is contacted with the liquid monomer or a mixture of the liquid monomers forming the second polymer or with a solution thereof in a solvent and, when constant weight is achieved, the monomer(s) are penetrated into the first polymer are polymerized and the polymeric material is cross-linked.

6.  A process of claim 5, **characterized in that** the first polymer and the monomer are contacted at a temperature between ambient temperature and 60°C for 1 to 336 hours, preferably at least for 48 hours, optionally in the presence of a solvent.

7.  A process of claim 6, **characterized in that** toluene, benzene, dioxane, or diethyl ether is used as a solvent.

8.  A process of claim 5, **characterized in that** prepolymerization is carried out in a first step, then in a second step the polymeric material is treated by high-energy irradiation.

9.  A process of claim 8, **characterized in that** prepolymerization is carried out by thermal treatment, UV radiation, or using a chemical initiator, and for the high-energy irradiation gamma radiation or electron radiation is used.

10. A process of claim 5, **characterized in that** the polymerization and cross-linking is carried out in one step using high-energy irradiation.

## Figure 1

(A)

(B)

(C)

(D)

Figure 2

**Flowchart a)**

| Sample preparation by cutting of the bulk | → | Soaking in liquid monomer | → | Wiping the surface until dry (optional step) | → | Prepolymer-ization | → | Wrapping | → | Treatment with high-energy radiation |

**Flowchart b)**

| Sample preparation by cutting of the bulk | → | Soaking in liquid monomer | → | Wipe up of the surface until dry | → | Wrapping | → | Treatment with high-energy radiation |

Figure 3

**Raman spectrum of the cross section of the polymeric material**

**Figure 4**

**Characteristic curve obtained by the image analysis of the Raman spectrum of the polymeric material**

Depth (μm)

**Figure 5**

**Change of the abrasion coefficient in the function of time**

Time (s)

**Europäisches Patentamt**
**European Patent Office**
**Office européen des brevets**

# EUROPEAN SEARCH REPORT

Application Number

EP 09 17 8527

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE WPI<br>Thomson Scientific, London, GB; AN 2007-643124<br>XP002571207<br>-& JP 2007 153945 A (UNIV NHON)<br>21 June 2007 (2007-06-21)<br>* abstract * | 1-2,4-5 | INV.<br>A61F2/02<br>A61L27/16<br>A61L27/34<br>C08J7/18 |
| X | US 2005/043789 A1 (WIDENHOUSE CHRISTOPHER W [US] ET AL)<br>24 February 2005 (2005-02-24)<br>* claims 1, 7-8, 14, 16, 19 * | 1,4-5,10 | |
| X | US 5 290 548 A (GOLDBERG EUGENE P [US] ET AL) 1 March 1994 (1994-03-01)<br>* claims 1,4, 5, 9, 11, 17; example 1 * | 1,4-6,10 | |
| X | US 3 372 100 A (ARTHUR CHARLESBY ET AL)<br>5 March 1968 (1968-03-05)<br>* claims; examples 1-3 * | 1,5-6,10 | |
| X | GB 844 231 A (DOW CHEMICAL CO)<br>10 August 1960 (1960-08-10)<br>* claims 1, 4, 10; example 1 C * | 1,3,5-6,10 | TECHNICAL FIELDS SEARCHED (IPC)<br>A61F<br>A61L<br>C08J |

The present search report has been drawn up for all claims

| Place of search | Date of completion of the search | Examiner |
|---|---|---|
| Munich | 4 March 2010 | Degrendel, Magali |

EPO FORM 1503 03.82 (P04C01)

## EP 2 196 171 A1

**ANNEX TO THE EUROPEAN SEARCH REPORT
ON EUROPEAN PATENT APPLICATION NO.**

EP 09 17 8527

This annex lists the patent family members relating to the patent documents cited in the above-mentioned European search report.
The members are as contained in the European Patent Office EDP file on
The European Patent Office is in no way liable for these particulars which are merely given for the purpose of information.

04-03-2010

| Patent document cited in search report | | | Publication date | Patent family member(s) | | Publication date |
|---|---|---|---|---|---|---|
| JP 2007153945 | A | | 21-06-2007 | NONE | | |
| US 2005043789 | A1 | | 24-02-2005 | NONE | | |
| US 5290548 | A | | 01-03-1994 | NONE | | |
| US 3372100 | A | | 05-03-1968 | GB | 866069 A | 26-04-1961 |
| GB 844231 | A | | 10-08-1960 | DE | 1544804 A1 | 04-09-1969 |
| | | | | DE | 1544805 A1 | 18-12-1969 |
| | | | | DE | 1218719 B | 08-06-1966 |
| | | | | FR | 1202891 A | 14-01-1960 |

EPO FORM P0459

For more details about this annex : see Official Journal of the European Patent Office, No. 12/82

**EP 2 196 171 A1**

**Patent documents cited in the description**

- DE 20321263 U1
- EP 1779877 A1
- US 6494917 B
- US 20070118227 A
- US 7186364 B
- US 5645594 A